(19) Europäisches Patentamt
European Patent Office
Office européen des brevets

(11) Numéro de publication : **0 325 540 B1**

(12) # FASCICULE DE BREVET EUROPEEN

(45) Date de publication du fascicule du brevet : **14.04.93 Bulletin 93/15**

(21) Numéro de dépôt : **89400162.7**

(22) Date de dépôt : **19.01.89**

(51) Int. Cl.$^5$ : **C07C 69/92,** C07C 67/14, C07C 327/20, C07C 235/42, C07D 265/30, A61K 31/235, A61K 31/255, A61K 7/48, A61K 7/06

(54) **Esters et thioesters aromatiques, leur procédé de préparation et leur utilisation en médecine humaine ou vétérinaire et en cosmétique.**

(30) Priorité : **20.01.88 LU 87109**

(43) Date de publication de la demande : **26.07.89 Bulletin 89/30**

(45) Mention de la délivrance du brevet : **14.04.93 Bulletin 93/15**

(84) Etats contractants désignés : **AT BE CH DE ES FR GB GR IT LI NL SE**

(56) Documents cités : **Eléments de la technique relevés: néant.**

(73) Titulaire : **CENTRE INTERNATIONAL DE RECHERCHES DERMATOLOGIQUES GALDERMA - CIRD GALDERMA**
**Sophia Antipolis**
**F-06560 Valbonne (FR)**

(72) Inventeur : **Shroot, Braham Villa 35 Hameaux de Val Bosquet Chemin de Val Bosquet**
**F-06600 Antibes (FR)**
Inventeur : **Eustache, Jacques**
**42, avenue Alphonse Daudet**
**F-06130 Grasse (FR)**
Inventeur : **Bernardon, Jean-Michel**
**21, Chemin Plan Bergier Le Rouret**
**F-06650 Nice (FR)**

(74) Mandataire : **Stalla-Bourdillon, Bernard et al**
**CABINET NONY & CIE 29, rue Cambacérès**
**F-75008 Paris (FR)**

EP 0 325 540 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux esters et thioesters aromatiques, leur procédé de préparation et leur utilisation en médecine humaine et vétérinaire et en cosmétique.

Ces nouveaux esters et thioesters aromatiques trouvent une application dans le traitement topique et systémique des affections dermatologiques liées à un désordre de la kératinisation (différenciation-prolifération) et d'affections dermatologiques, ou autres, à composantes inflammatoires et/ou immunoallergiques et dans les maladies de dégénérescence du tissu conjonctif, et présentent une activité antitumorale. En outre, ces dérivés peuvent être utilisés dans le traitement de l'atopie, qu'elle soit cutanée ou respiratoire et du psoriasis rhumatoïde.

Ils trouvent également une application dans le domaine ophtalmologique, notamment dans le traitement des cornéopathies.

On connaît déjà pour ces applications des dérivés "benzamido différines" tels que décrits dans le brevet luxembourgeois n° 86 258.

Il a maintenant été découvert de manière inattendue qu'en remplaçant l'atome d'azote de la liaison amido par un atome d'oxygène ou de soufre et en variant certains autres substituants des benzamido-différines, on arrive à des esters et thioesters aromatiques ayant une bonne activité dans les domaines d'application ci-dessus indiqués.

Les esters et thioesters selon l'invention peuvent être représentés par la formule générale suivante :

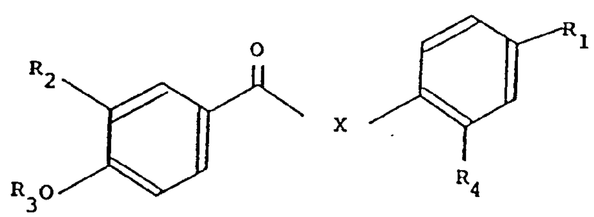

(I)

dans laquelle:

X représente un atome d'oxygène ou de soufre,

$R_1$ représente: $-CH_2OH$ , $-CH(OH)-Ch_3$ , $-COOR_5$ ou

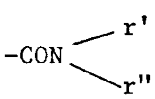

$R_5$ représentant un atome d'hydrogène, un radical alkyle ou alkényle inférieur ou un radical mono ou polyhydroxyalkyle,

r' et r" représentant un atome d'hydrogène, un radical alkyle inférieur, un radical mono ou polyhydroxyalkyle, un radical aryle ou benzyle éventuellement substitué(s), un reste d'aminoacide ou de sucre aminé ou pris ensemble forment un hétérocycle,

$R_2$ représente un radical alkyle $\alpha$, $\alpha'$ disubstitué ayant de 4 à 12 atomes de carbone ou un radical cycloalkyle mono ou polycyclique ayant de 5 à 12 atomes de carbone dont le carbone de liaison est trisubstitué,

$R_3$ représente un atome d'hydrogène, un radical alkyle inférieur, ou le radical $-Si(CH_3)_2 R'_3$, $R'_3$ représentant un radical alkyle inférieur,

et $R_4$ représente un atome d'hydrogène ou un radical alkyle inférieur,

et les sels desdits esters et thioesters aromatiques de formule (I) lorsque $R_5$ représente un atome d'hydrogène.

Lorsque les composés selon l'invention se présentent sous forme de sels, il peut s'agir de sels d'un métal alcalin ou alcalino-terreux ou encore de zinc ou d'une amine organique.

Par radical alkyle inférieur, on doit entendre un radical ayant de 1 à 6 atomes de carbone notamment les radicaux méthyle, éthyle, isopropyle, butyle et tertiobutyle.

Par radical alkyle $\alpha,\alpha'$ disubstitué ayant de 4 à 12 atomes de carbone on doit notamment entendre un radical tert-butyle, 1,1-diméthyl propyle, 1-méthyl 1-éthyl propyle, 1-méthyl 1-éthyl hexyle ou 1,1-diméthyl décyle.

Par radical monohydroxyalkyle, on doit entendre un radical ayant 2 ou 3 atomes de carbone, notamment un radical 2-hydroxyéthyle, 2-hydroxypropyle ou 3-hydroxypropyle.

Par radical polyhydroxyalkyle, on doit entendre un radical contenant de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on doit entendre un radical phényle éventuellement substitué par un atome d'halogène, un hydroxyle ou une fonction nitro.

Par radical cycloalkyle mono ou polycyclique ayant de 5 à 12 atomes de carbone dont le carbone de liaison est trisubstitué on doit entendre le radical 1-méthyl cyclohexyle ou 1-adamantyle.

Par reste d'aminoacide on doit entendre un reste dérivant par exemple de la lysine ou de la glycine.

Par reste d'un sucre aminé, on doit entendre un reste dérivant par exemple de glucosamine, de galactosamine ou de mannosamine.

Lorsque les radicaux r′ et r″ pris ensemble forment un hétérocycle, celui-ci est de préférence un radical pipéridino, pipérazino, morpholino, pyrrolidino ou (2-hydroxyéthyl)-4-pipérazino.

Parmi les composés de formule (I) ci-dessus, on peut notamment citer les suivants:

- l'acide 4-[3-(1-adamantyl)-4-méthoxybenzoyloxy] benzoïque,
- l'acide 4-[3-(1-adamantyl)-4-méthoxybenzoylthio] benzoïque,
- l'acide 4-[3-(1-méthylcyclohexyl)-4-méthoxybenzoyloxy] benzoïque,
- l'acide 4-[3-(1-méthylcyclohexyl)-4-méthoxybenzoylthio] benzoïque,
- l'acide 4-[3-(1,1-diméthyldécyl)-4-méthoxybenzoyloxy] benzoïque,
- l'acide 4-[3-(1,1-diméthyldécyl)-4-méthoxybenzoylthio] benzoïque,
- l'acide 4-[3-(1-adamantyl)-4-hydroxybenzoyloxy] benzoïque,
- l'acide 4-[3-(1-adamantyl)-4-hydroxybenzoylthio] benzoïque,
- le 4-[3-(1-adamantyl)-4-méthoxybenzoyloxy] benzoate d'éthyle,
- le 4-[3-(1-adamantyl)-4-méthoxybenzoylthio] benzoate d'éthyle,
- le N-éthyl 4-[3-(1-adamantyl)-4-méthoxybenzoyloxy] benzamide,
- le morpholide de l'acide 4-[3-(1-adamantyl)-4 méthoxybenzoylthio] benzoïque,
- le 4-[3-(1-adamantyl)-4-méthoxybenzoyloxy] benzoate d'allyle.
- l'acide 4-[3-tert-butyl-4-méthoxybenzoylthio] benzoïque,
- le 4-[3-(1-adamantyl)-4-méthoxybenzoylthio] benzoate de méthyle,
- le N-éthyl 4-[3-(1-adamantyl)-4-méthoxybenzoylthio] benzamide,
- le 4-[3-(1-adamantyl)-4-méthoxybenzoylthio] benzoate de 2-hydroxyéthyle,
- l'acide 4-[3-(1-adamantyl)-4-tert-butyldiméthylsilyloxy benzoylthio]benzoïque,
- le 4-[3-(1-adamantyl)-4-hydroxybenzoyloxy] benzoate d'allyle,
- l'acide 3-méthyl-4-[3-(1-adamantyl)-4-méthoxybenzoyloxy] benzoïque,
- l'aldéhyde 4-[3-(1-adamantyl)-4-méthoxybenzoyloxy] benzoïque,
- l'alcool 4-[3-(1-adamantyl)-4-méthoxybenzoyloxy] benzylique, et
- l'acide 3-tert-butyl-4-[3-(1-adamantyl-)4-méthoxybenzoyloxy] benzoïque.

La présente invention a également pour objet le procédé de préparation des composés de formule (I) selon le schéma réactionnel suivant:

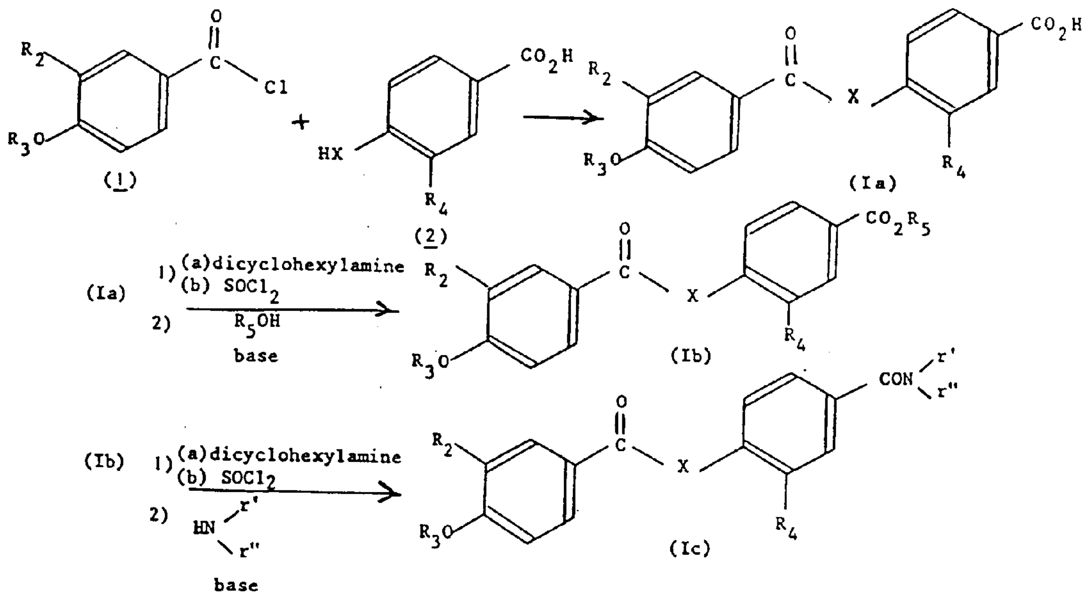


L'étape principale de cette préparation consiste à faire réagir en milieu anhydre, dans un solvant organique tel que le tétrahydrofuranne ou le chlorure de méthylène contenant une amine tertiaire (pyridine ou triéthylamine), une forme activée d'un acide benzoïque substitué, par exemple un chlorure d'acide (1), ou un anhydride mixte, sur un acide p-hydroxy ou p-thio benzoïque éventuellement substitué en position 3 (2), la réaction étant conduite à température ambiante et sous agitation.

Les acides (Ia) ainsi obtenus peuvent être convertis de manière connue en chlorure d'acide correspondant qui, traité par un alcool ($R_5OH$)
ou une amine

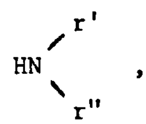

donne l'ester (Ib) ou l'amide (Ic) correspondant.

Les composés de formule (Ia) dans laquelle X représente un atome d'oxygène peuvent également être préparés selon le schéma réactionnel suivant:

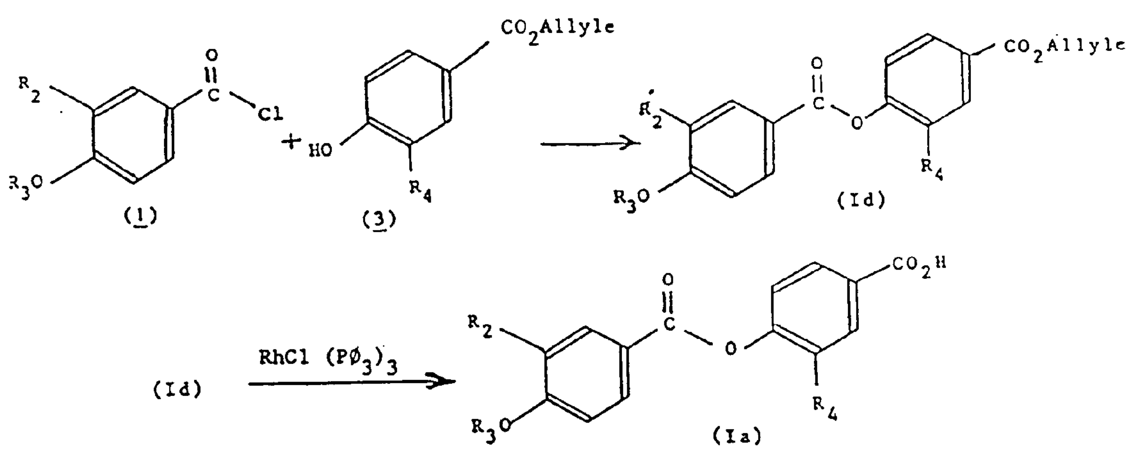

L'action du chlorure d'acide (1) avec un p-hydroxybenzoate d'allyle (3), éventuellement substitué en position 3, en présence d'une amine tertiaire telle que la pyridine ou la triéthylamine, conduit aux esters allyliques (Id). Le passage à l'acide libre peut être effectué au moyen d'un catalyseur tel que certains complexes de métaux de transition par exemple le tris (triphénylphosphine) rhodium (1) chlorure ou le tétrakis (triphénylphosphine) palladium (o) en présence d'une amine secondaire.

La présente invention a également pour objet à titre de médicament les composés de formule (I) telle que définie ci-dessus.

Les composés selon l'invention présentent une bonne stabilité à la lumière et à l'oxygène.

Ces composés présentent une bonne activité dans le test d'inhibition de l'ornithine décarboxylase après induction par "tape stripping" chez le rat nu. (M. Bouclier et coll, DERMATOLOGICA 169 N°4, 1984). Ce test est admis comme mesure de l'action inhibitrice de certains composés sur les phénomènes de prolifération cellulaire.

Ils présentent également une bonne activité dans le test de différenciation des cellules de tératocarcinome embryonnaire de la souris (cellules F9) (Cancer Research 43, p5268, 1983).

Enfin, les nouveaux composés se caractérisent par l'introduction dans la structure chimique d'une liaison ester ou thioester hautement sensible à l'action de diverses estérases in vivo, ce qui conduit à l'inactivation rapide des molécules par conversion en fragments biologiquement inactifs.

Les composés selon l'invention conviennent particulièrement bien dans les domaines de traitement suivants :

1) pour traiter les affections dermatologiques liées à un désordre de la kératinisation portant sur la différenciation et sur la prolifération notamment pour traiter les acnés vulgaires, comédoniennes, polymorphes, les acnés nodulo kystiques, conglobata, les acnés séniles, les acnés secondaires telles que l'acné solaire, médicamenteuse, professionnelle ;

2) pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen ;

3) pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriasique, ou encore l'atopie cutanée, telle que l'eczéma, ou l'atopie respiratoire ; les composés peuvent également être utilisés dans certaines affections inflammatoires ne présentant pas de trouble de la kératinisation.

4) pour traiter toutes les proliférations dermiques ou épidermiques qu'elles soient bénignes ou malignes, qu'elles soient d'origine virale telles que verrues vulgaires, les verrues planes et l'épidermodysplasie verruciforme, les proliférations pouvant également être induites par les ultra-violets notamment dans le cas des épithélioma baso et spino cellulaires ;

5) pour traiter d'autres désordres dermatologiques tels que les dermatoses bulleuses et les maladies du collagène ;

6) pour traiter certains troubles ophtalmologiques, notamment les cornéopathies ;

7) pour lutter contre le vieillissement de la peau, qu'il soit photoinduit ou non ;

8) pour prévenir ou guérir les stigmates de l'atrophie épidermique et/ou dermique induite par les corticostéroïdes locaux ou systémiques, ou tout autre forme d'atrophie cutanée.

La présente invention a donc également pour objet des compositions médicamenteuses contenant au moins un composé de formule (I) tel que défini ci-dessus, ou un de ses sels.

La présente invention a donc aussi pour objet une nouvelle composition médicamenteuse, destinée notamment au traitement des affections susmentionnées, caractérisée par le fait qu'elle comporte, dans un support pharmaceutique acceptable, au moins un composé de formule (I) et/ou un de ses sels.

Les composés selon l'invention sont généralement administrés à une dose journalière d'environ 0,01mg/kg à 100mg/kg de poids corporel.

Comme support des compositions, on peut utiliser tout support conventionnel, le composé actif se trouvant soit à l'état dissous, soit à l'état dispersé dans le véhicule.

L'administration peut être effectuée par voie entérale, parentérale, topique ou oculaire. Par voie entérale, les médicaments peuvent se présenter sous forme de comprimés, de gélules, de dragées, de sirops, de suspensions, de solutions, de poudres, de granulés, d'émulsions. Par voie parentérale, les compositions peuvent se présenter sous forme de solutions ou suspensions pour perfusion ou pour injection.

Par voie topique, les compositions pharmaceutiques à base des composés selon l'invention se présentent sous forme d'onguents, de teintures, de crèmes, de pommades, de poudres, de timbres, de tampons imbibés, de solutions, de lotions, de gels, de sprays ou encore de suspensions.

Ces compositions par voie topique peuvent se présenter soit sous forme anhydre, soit sous forme aqueuse

selon l'indication clinique.

Par voie oculaire, ce sont principalement des collyres.

Ces compositions contiennent au moins un composé de formule (1) tel que défini ci-dessus ou un de ses sels, à une concentration de préférence comprise entre 0,0001 et 5% par rapport au poids total de la composition.

Les composés de formule (I), selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches.

La présente invention vise donc également une composition cosmétique contenant, dans un support cosmétiquement acceptable, au moins un composé de formule (I) ou un de ses sels, cette composition se présentant notamment sous forme de lotion, gel, savon ou shampooing.

La concentration en composé de formule (I), dans les compositions cosmétiques est comprise entre 0,0001 et 0,1% en poids et de préférence entre 0,001 et 0,01% en poids.

Les compositions médicamenteuses et cosmétiques selon l'invention peuvent contenir des additifs inertes ou même pharmacodynamiquement ou cosmétiquement actifs et notamment: des agents hydratants comme la thiamorpholinone et ses dérivés ou l'urée; des agents antiséborrhéiques ou antiacnéiques, tels que la S-carboxyméthylcystéine, la S-benzyl-cystéamine ,leurs sels et leur dérivés, la tioxolone ou le peroxyde de benzoyle; des antibiotiques comme l'érythromycine et ses esters, la néomycine, les tétracyclines ou les polyméthylène-4,5 isothiazolinones-3; des agents favorisant la repousse des cheveux, comme le "Minoxidil" (2,4-diamino-6- pipéridino-pyrimidine-3-oxyde) et ses dérivés, le Diazoxide (7-chloro 3-méthyl 1,2,4-benzothiadiazine 1,1-dioxyde) et le Phénytoïn (5-5-diphényl- imidazolidine 2,4-dione); des agents anti-inflammatoires stéroïdiens et non stéroïdiens; des caroténoïdes et, notamment le β-carotène; des agents anti-psoriasiques tels que l'anthraline et ses dérivés et les acides eicosatétraynoïque-5,8,11,14 et triynoïque-5,8,11, leurs esters et leurs amides.

Les compositions selon l'invention peuvent également contenir des agents d'amélioration de la saveur, des agents conservateurs, des agents stabilisants, des agents régulateurs d'humidité, des agents régulateurs de pH, des agents modificateurs de pression osmotique, des agents émulsionnants, des filtres UV-A et UV-B, des anti-oxydants tels que l'$\alpha$-tocophérol, le butylhydroxyanisole ou le butylhydroxy toluène.

On va maintenant donner, à titre d'illustration et sans aucun caractère limitatif, plusieurs exemples de préparation des composés actifs de formule (I) selon l'invention ainsi que des exemples de compositions les contenant.

## EXEMPLE 1

### Préparation du 4-[3-(1-adamantyl)-4-méthoxybenzoyloxy] benzoate d'allyle

2,86g (10mmoles) d'acide 3-(1-adamantyl)-4-méthoxybenzoïque décrit dans le brevet français N° 85 13747 (2.570.377) à l'exemple 26 et 10ml de chlorure de thionyle ($SOCl_2$) sont chauffés à reflux jusqu'à cessation du dégagement gazeux. On évapore à sec, reprend par 20ml de tétrahydrofuranne (THF) et la solution ainsi obtenue est ajoutée goutte à goutte à une solution de p-hydroxybenzoate d'allyle (1,78g, 10mmoles) et de triéthylamine (1,5ml, 11mmoles), dans le THF (50ml).

On agite 4h à 20°C, jette dans l'eau, extrait à l'éther, sèche sur sulfate de magnésium, évapore les solvants et chromatographie le résidu sur colonne de silice (éluant $CH_2Cl_2$-hexane 1/1). On obtient ainsi 3,0g (68%) du produit attendu ayant un point de fusion de: 109-110°C.

## EXEMPLE 2

### Préparation de l'acide 4-[3-(1-adamantyl)-4-méthoxybenzoyloxy] benzoïque

2,8g (6,2mmoles) d'ester préparé à l'exemple 1 sont dissous dans 100ml d'un mélange d'éthanol et eau (9/1). 75mg de complexe tris (triphényl- phosphine) rhodium chlorure sont ajoutés et on chauffe à reflux 12h. On refroidit, filtre le solide que l'on dissout dans un mélange de dichlorométhane et de THF (9/1). On lave à l'eau, sèche ($MgSO_4$) puis évapore les solvants.

Le solide est repris par 50ml d'éther éthylique pour donner 1g (40%) d'acide 4-[3-(1-adamantyl)-4-méthoxybenzoyloxy] benzoïque de point de fusion: 265-267°C.

EXEMPLE 3

Préparation de l'acide 4-[3-(1-adamantyl)-4-méthoxybenzoylthio] benzoïque

Dans un ballon, on introduit 1,7g (11mmoles) d'acide 4-mercaptobenzoïque, 20ml de pyridine et ajoute goutte à goutte une solution de 3,4g (11,17 mmoles) de chlorure de l'acide 3-(1-adamantyl)-4-méthoxybenzoïque dans 50 ml de dichlorométhane. On agite à température ambiante 8 heures, évapore à sec, reprend par 100ml d'eau, acidifie à pH5 avec de l'acide chlorhydrique 1N. Le solide est filtré, lavé à l'eau et séché à 60°C sous vide puis recristallisé dans du dioxanne. On obtient 3,3g (75%) d'acide 4-[3-(1-adamantyl-4-méthoxybenzoylthio] benzoïque de point de fusion : 264°C.

EXEMPLE 4

Préparation de l'acide 4-[3-(1-méthylcyclohexyl)-4-méthoxybenzoylthio] benzoïque

(a) 4-bromo-2-(1-méthylcyclohexyl)phénol.

Dans un ballon, on introduit 11,42g (100 mmoles) de 1-méthylcyclohexanol, 50ml d'heptane et 270 µl d'acide sulfurique concentré. On ajoute goutte à goutte 10,8ml (117 mmoles) d'anhydride acétique et agite à température ambiante pendant 18 heures. On additionne ensuite 2,7ml (50 mmoles) d'acide sulfurique concentré et par petites portions 17,3g (100 mmoles) de 4-bromophénol. On agite à température ambiante pendant 24 heures, évapore à sec, reprend par 200 ml d'eau, ajuste à pH7 avec du bicarbonate de sodium et extrait avec de l'éther éthylique. On décante la phase organique, sèche sur sulfate de magnésium et évapore. Le résidu est purifié par chromatographie sur colonne de silice en utilisant comme éluant un mélange de dichlorométhane et d'hexane (40/60). On évapore les solvants et obtient 8,97g (33%) de 4-bromo-2-(1-méthylcyclohexyl)phénol sous la forme d'une huile jaunâtre.

(b) 4-bromo-2-(1-méthylcyclohexyl)anisole.

Le 4-bromo-2-(1-méthylcyclohexyl)phénol (9,26g, 34,4 mmoles) est dissous dans 50ml de THF. On refroidit à 0°C et ajoute par petites portions 1,14g (37,8 mmoles) d'hydrure de sodium (80% dans l'huile). On agite trente minutes à température ambiante et ajoute goutte à goutte 5,37g (37,8 mmoles) d'iodure de méthyle. On continue l'agitation pendant 16 heures, ajoute de l'eau (300ml) et extrait par de l'éther éthylique (3x300ml). La phase organique est décantée et lavée avec une solution saturée de bicarbonate de sodium, puis une solution saturée de chlorure de sodium. On sèche ($MgSO_4$), filtre et évapore les solvants. Le résidu est purifié par chromatographie sur colonne de silice, élué par un mélange composé de dichlorométhane et d'hexane (20/80). On obtient ainsi 9g (92%) de 4-bromo-2-(1-méthylcyclohexyl)anisole sous forme d'une huile incolore.

(c) Acide 3-(1-méthylcyclohexyl)-4-méthoxybenzoïque.

Le composé obtenu en 4(b) (9g, 31,8 mmoles) est dissous dans 50 ml de THF sec. La solution obtenue est ajoutée goutte à goutte sur du magnésium (850mg, 35 mmoles) et un cristal d'iode. Après l'introduction des 5 premiers millilitres, on chauffe à reflux et maintient celui-ci 15 mn après que l'addition soit terminée. On refroidit alors à -40°C et fait passer un courant de $CO_2$ pendant une heure, puis jette le mélange dans de l'acide chlorhydrique 6N, extrait avec de l'éther éthylique (3x300ml). La phase organique est décantée, lavée à l'eau jusqu'à neutralité, séchée ($MgSO_4$), et évaporée. Le résidu obtenu est trituré dans de l'hexane, filtré et séché. On obtient ainsi 6,50g (82%) d'acide 3-(1-méthylcyclohexyl)-4-méthoxybenzoïque de point de fusion : 199°C.

(d) Chlorure de l'acide 3-(1-méthylcyclohexyl)-4-méthoxybenzoïque.

1,24g (5 mmoles) d'acide 3-(1-méthylcyclohexyl) -4-méthoxybenzoïque et 15ml de chlorure de thionyle sont chauffés à reflux jusqu'à cessation du dégagement gazeux. On évapore à sec et obtient 1,32g (100%) de chlorure de l'acide 3-(1-méthylcyclohexyl)-4-méthoxybenzoïque brut qui est utilisé tel quel pour la suite de la synthèse.

(e) Acide 4-[3-(1-méthylcyclohexyl)-4-méthoxybenzoylthio] benzoïque.

Dans un ballon, on introduit 771mg (5 mmoles) d'acide 4-mercaptobenzoïque, 8ml de pyridine et ajoute

goutte à goutte une solution de 1,32g (5 mmoles) du chlorure d'acide obtenu en 4(d) dissous dans 20ml de dichlorométhane et agite à température ambiante pendant 5 heures. On évapore à sec, reprend par l'eau, acidifie à pH5 avec de l'acide chlorhydrique 1N et extrait à l'éther éthylique (500ml). On décante la phase organique, sèche sur sulfate de magnésium et évapore à sec. Le résidu obtenu est purifié par chromatographie sur colonne de silice, élué par un mélange composé de dichlorométhane et d'éther éthylique (80/20). On obtient ainsi 1,55g (81%) d'acide 4-[3-(méthylcyclohexyl)-4-méthoxybenzoylthio] benzoïque de point de fusion : 216-218°C.

EXEMPLE 5

Préparation de l'acide 4-[3-(1,1-diméthyldécyl)-4-méthoxybenzoylthio] benzoïque

Le chlorure de l'acide 3-(1,1-diméthyldécyl)-4-méthoxybenzoïque brut, préparé à partir de 1,6g (5 mmoles) d'acide 3-(1,1-diméthyldécyl)-4-méthoxybenzoïque, décrit dans la demande de brevet Européen N° 0.232.199 à l'exemple 11, est dissous dans 20ml de dichlorométhane. La solution est ajoutée goutte à goutte sur un mélange de 771mg (5 mmoles) d'acide 4-mercaptobenzoïque et de 8ml de pyridine. On agite à température ambiante pendant 8 heures, évapore à sec, reprend par l'eau, acidifie à pH5 avec de l'acide chlorhydrique 1N et extrait avec de l'éther éthylique (500ml). On décante la phase organique, lave à l'eau, sèche sur sulfate de magnésium et évapore à sec. Le résidu obtenu est purifié par chromatographie sur colonne de silice, en utilisant comme éluant de l'éther éthylique. On obtient ainsi 1,92g (84%) d'acide 4-[3-(1,1-diméthyldécyl)-4-méthoxybenzoylthio] benzoïque, de point de fusion : 186-187°C.

EXEMPLE 6

Préparation de l'acide 4-[3-tert-butyl-4-méthoxybenzoylthio] benzoïque

Le chlorure de l'acide 3-(tert-butyl)-4-méthoxybenzoïque brut, préparé à partir de 1,04g (5 mmoles) d'acide 3-(tert-butyl) 4-méthoxybenzoïque, décrit dans le brevet français N° 85 13747 (2.570.377) à l'exemple 35, est dissous dans 15ml de dichlorométhane. La solution est ajoutée goutte à goutte sur un mélange de 771mg (5 mmoles) d'acide 4-mercaptobenzoïque et de 8ml de pyridine. On agite à température ambiante pendant 8 heures, évapore à sec, reprend par l'eau, acidifie à pH5 avec de l'acide chlorhydrique 1N et extrait par de l'éther éthylique. On décante la phase organique, lave à l'eau, sèche sur sulfate de magnésium et évapore à sec. Le résidu obtenu est purifié par chromatographie sur colonne de silice (éluant éther éthylique). On évapore le solvant et obtient 530mg (32%) d'acide 4-[3-tert-butyl-4-méthoxybenzoylthio]benzoïque, de point de fusion : 220-221°C.

EXEMPLE 7

Préparation du 4-[3-(1-adamantyl)-4-méthoxybenzoylthio] benzoate de méthyle

(a) Chlorure de l'acide 4-[3-(1-adamantyl)-4-méthoxybenzoylthio] benzoïque

Dans un ballon, on introduit 3,80g (9 mmoles) d'acide 4-[3-(1-adamantyl)-4-méthoxybenzoylthio] benzoïque, 40ml de dichlorométhane, 1,8ml (9 mmoles) de dicyclohexylamine, et agite pendant une heure. A la solution ainsi obtenue, on ajoute 720 µl (9,9 mmoles) de chlorure de thionyle et agite à température ambiante pendant 2 heures. On évapore à sec, reprend par 500ml d'éther éthylique, filtre le chlorure de dicyclohexylammonium puis évapore le solvant. On obtient ainsi 3,97g (100%) de chlorure de 4 [3-(1-adamantyl)-4-méthoxybenzoylthio] benzoyle brut qui est utilisé tel quel pour la suite de la synthèse.

(b) 4-[3-(1-adamantyl)-4-méthoxybenzoylthio] benzoate de méthyle.

Une solution de 1,28g (2,9 mmoles) du chlorure d'acide obtenu à l'exemple 7(a) dans 30ml de THF est ajoutée goutte à goutte à un mélange de 240 µl (5,8 mmoles) d'alcool méthylique et de 404 µl (2,9 mmoles) de triéthylamine dans 20ml de THF. Le mélange est agité pendant 8 heures puis jeté dans de l'eau et extrait avec de l'éther éthylique (500ml). La phase organique est décantée, lavée à l'eau, séchée sur sulfate de magnésium puis évaporée. Le résidu ainsi obtenu est purifié par chromatographie sur colonne de silice, élué avec un mélange de dichlorométhane et d'hexane (40-60). On évapore les solvants et obtient 890mg (70%) d'ester attendu de point de fusion : 138-140°C.

EXEMPLE 8

Préparation du N-éthyl 4-[3-(1-adamantyl)-4-méthoxybenzoylthio] benzamide.

Une solution de 1,28g (2,9 mmoles) du chlorure d'acide obtenu à l'exemple 7(a) dans 30ml de THF est ajoutée goutte à goutte à une solution de 395 µl (5,8 mmoles) d'éthylamine dans 20ml de THF. Le mélange est agité pendant 4 heures puis jeté dans l'eau et extrait avec de l'éther éthylique (500ml). La phase organique est décantée, lavée à l'eau, séchée sur sulfate de magnésium et évaporée. Le résidu obtenu est purifié par chromatographie sur colonne de silice, élué avec un mélange d'éther éthylique et de dichlorométhane (20/80). On évapore les solvants et obtient 820mg (61%) d'amide attendu de point de fusion : 185-186°C.

EXEMPLE 9

Préparation du morpholide de l'acide 4-[3(1-adamantyl)-4-méthoxybenzoylthio] benzoïque

Une solution de 1,28g (2,9 mmoles) du chlorure d'acide obtenu à l'exemple 7(a) dans 30ml de THF est ajouté goutte à goutte à un mélange de 510 µl (5,8 mmoles) de morpholine dans 30ml de THF. Le mélange est agité pendant 16 heures puis jeté dans l'eau et extrait avec de l'éther éthylique (800ml). La phase organique est décantée, lavée à l'eau, séchée sur sulfate de magnésium et évaporée à sec. Le résidu obtenu est trituré dans 30ml d'éther éthylique, filtré et séché. On recueille 960mg (68%) d'amide de point de fusion : 178-180°C.

EXEMPLE 10

Préparation du 4-[3-(1-adamantyl)-4-méthoxybenzoylthio] benzoate de 2-hydroxyéthyle

Une solution de 1,28g (2,9 mmoles) du chlorure d'acide obtenu à l'exemple 7(a) dans 30ml de THF est ajoutée goutte à goutte à un mélange de 1,62ml (29 mmoles) d'éthylène glycol et de 235 µl (2,9 mmoles) de pyridine dans 20ml de THF. Le mélange est agité pendant 16 heures puis jeté dans l'eau et extrait avec de l'éther éthylique (500ml). La phase organique est décantée, lavée à l'eau, séchée sur sulfate de magnésium et évaporée. Le résidu obtenu est recristallisé dans un mélange d'éther isopropylique et de cyclohexane (80/20). On recueille 783mg (58%) de l'ester attendu de point de fusion : 115-117°C.

EXEMPLE 11

Préparation de l'acide 4-[3-(1-adamantyl)-4-tert-butyldiméthylsilyloxybenzoylthio] benzoïque

Le chlorure de l'acide 3-(1-adamantyl)-4-tert -butyldiméthylsilyloxybenzoïque brut, préparé à partir de 3,8g (9,95 mmoles) de l'acide correspondant, décrit dans la demande de brevet européen N° 0.232.199 à l'exemple 3, est dissous dans 40ml de dichlorométhane. La solution est ajoutée goutte à goutte sur un mélange de 1,54g (9,95 mmoles) d'acide 4-mercaptobenzoïque et de 15ml de pyridine. On agite à température ambiante 12 heures, évapore à sec, reprend par l'eau, acidifie à pH5 avec de l'acide chlorhydrique 1N. Le solide est filtré, lavé à l'eau, trituré avec 50ml d'éther éthylique et séché à 60°C sous vide. On recueille 5g (96%) d'une poudre blanche de point de fusion : 232-233°C.

EXEMPLE 12

Préparation de l'acide 4-[3-(1-adamantyl)-4-hydroxy benzoylthio] benzoïque

Dans un ballon, on introduit 3,66g (7 mmoles) de l'acide obtenu à l'exemple 11 et 50ml de THF. On ajoute goutte à goutte 7,7ml (7,7 mmoles) de fluorure de tétrabutylammonium (1M dans le THF) et agite à température ambiante pendant une heure. On jette le milieu réactionnel dans l'eau, extrait avec du dichlorométhane, décante la phase organique, sèche sur sulfate de magnésium et évapore les solvants. On triture le solide obtenu dans 100ml d'acétate d'éthyle à reflux, refroidit et filtre. On obtient ainsi 1,28g (45%) d'acide 4-[3-(1-adamantyl)-4-hydroxybenzoylthio] benzoïque, de point de fusion : 272-274°C.

EXEMPLE 13

Préparation du 4-[3-(1-adamantyl)-4-hydroxybenzoyloxy] benzoate d'allyle

(a) 4-[3-(1-adamantyl)-4-tert-butyldiméthylsilyloxybenzoyloxy] benzoate d'allyle.

Le chlorure de l'acide 3-(1-adamantyl)-4-tert-butyldiméthylsilyloxybenzoïque brut, préparé à partir de 3,7g (9,6 mmoles) de l'acide correspondant, est dissous dans 35ml de THF. La solution est ajoutée goutte à goutte sur un mélange de 1,72g (9,6 mmoles) de 4-hydroxybenzoate d'allyle, 1,5ml (10,6 mmoles) de triéthylamine et 25ml de THF. On agite à température ambiante pendant 12 heures, verse le milieu réactionnel dans l'eau, extrait avec de l'éther éthylique. On décante la phase organique, lave à l'eau, sèche sur sulfate de magnésium et évapore. Le résidu obtenu est purifié par chromatographie sur colonne de silice, en utilisant comme éluant un mélange de dichlorométhane et d'hexane (50/50). On recueille ainsi 3,96g (75%) d'ester allylique de point de fusion : 118-120°C.

(b) 4-[3-(1-adamantyl)-4-hydroxybenzoyloxy]benzoate d'allyle.

Dans un ballon, on introduit 3,91g (7,15 mmoles) de l'ester d'allyle obtenu à l'exemple 13(a) et 50ml de THF. On ajoute goutte à goutte 7,9ml (7,9 mmoles) de fluorure de tétrabutylammonium (1M dans le THF) et agite à température ambiante pendant deux heures. On jette le milieu réactionnel dans l'eau, extrait avec du dichlorométhane, décante la phase organique, sèche sur sulfate de magnésium et évapore. Le résidu obtenu est purifié par chromatographie sur colonne de silice, élué par un mélange de dichlorométhane et d'hexane (80-20). Après évaporation des solvants, on obtient 2,88g (93%) de 4-[3-(1-adamantyl)-4-hydroxybenzoyloxy] benzoate d'allyle de point de fusion : 175-177°C.

EXEMPLE 14

Préparation de l'acide 4-[3-(1-adamantyl-)4-hydroxybenzoyloxy] benzoïque

Dans un ballon, on introduit 2,16g (5 mmoles) de l'ester obtenu dans l'exemple 13(b) et 30ml de THF. Sous courant d'azote, on ajoute 290mg de tétrakis (triphénylphosphine) palladium (0) puis introduit goutte à goutte 4,35ml (50 mmoles) de morpholine. On agite à température ambiante deux heures, évapore à sec, triture le résidu dans l'éther et filtre le solide. On introduit le solide dans 100ml d'eau, acidifie à pH1, extrait avec 800ml d'éther éthylique. On décante la phase organique, lave à l'eau, sèche sur sulfate de magnésium et évapore. Le résidu est trituré dans 50ml d'éther éthylique, filtré et séché. On obtient 1,05g (54%) d'acide attendu de point de fusion : 269-271°C.

EXEMPLE 15

Préparation de l'acide 3-méthyl-4[3-(1-adamantyl)-4-méthoxybenzoyloxy] benzoïque

(a) 4-hydroxy-3-méthylbenzoate d'allyle.

Dans un ballon, on introduit 9,5g (62 mmoles) d'acide 4-hydroxy-3-méthylbenzoïque et 70ml d'alcool allylique. On ajoute 1,8ml d'acide sulfurique concentré et chauffe à 100°C durant 8 heures. On évapore à sec, ajoute 200ml d'eau, neutralise avec du bicarbonate de sodium et extrait avec du dichlorométhane. La phase organique est décantée, lavée à l'eau, séchée sur sulfate de magnésium et évaporée. Le résidu obtenu est purifié par simple filtration sur silice (éluant dichlorométhane). Après évaporation des solvants, on obtient 8,76g (92%) de l'ester attendu de point de fusion : 66-68°C.

(b) 3-méthyl-4-[3-(1-adamantyl)-4-méthoxybenzoyloxy] benzoate d'allyle.

Dans un ballon, on introduit 1,92g (10mmoles) de l'ester obtenu en 15(a), 1,5ml (11 mmoles) de triéthylamine et 50ml de THF. On ajoute goutte à goutte une solution de 3,04g (10 mmoles) de chlorure de l'acide 3-(1-adamantyl)-4-méthoxybenzoïque dans 20ml de THF et agite pendant 24 heures à température ambiante. Le milieu réactionnel est jeté dans l'eau et extrait avec de l'éther éthylique. La phase organique est décantée, lavée à l'eau bicarbonatée puis à l'eau, séchée sur sulfate de magnésium et évaporée. Le résidu obtenu est purifié par chromatographie sur colonne de silice en éluant avec un mélange de dichlorométhane et d'hexane

(50/50). On recueille 3g (65%) de 3-méthyl-4-[3-(1-adamantyl)-4-méthoxybenzoyloxy]benzoate d'allyle de point de fusion 138-139°C.

(c) Acide 3-méthyl-4[3-(1-adamantyl)-4-méthoxybenzoyloxy] benzoïque.

2,3g (5 mmoles) d'ester préparé à l'exemple 15(b) sont dissous dans 50ml de THF. Sous courant d'azote, on ajoute 78,3mg de tétrakis(triphénylphosphine)palladium(0) puis introduit goutte à goutte 4,4ml (50 mmoles) de morpholine. On agite à température ambiante 2 heures, évapore à sec, triture le résidu dans l'éther éthylique et filtre le solide. On introduit le solide dans 100ml d'eau, acidifie à pH1, extrait avec un mélange d'éther éthylique et de THF (3/1). On décante la phase organique, lave à l'eau, sèche sur sulfate de magnésium et évapore. Le résidu est trituré dans 50ml d'éther éthylique, filtré et séché. On obtient 1,6g (70%) d'acide attendu de point de fusion : 276-277°C.

EXEMPLE 16

Préparation de l'aldéhyde 4-[3-(1-adamantyl)-4-méthoxy benzoyloxy]benzoïque

Dans un ballon, on introduit 1,22g (10 mmoles) de 4-hydroxybenzaldéhyde, 1,53ml (11 mmoles) de triéthylamine et 50ml de THF. On ajoute goutte à goutte une solution de 3,04g (10 mmoles) de chlorure de l'acide 3-(1-adamantyl)-4-méthoxybenzoïque dans 20ml de THF et agite pendant 18 heures à température ambiante. Le milieu réactionnel est jeté dans l'eau et extrait avec de l'éther éthylique. La phase organique est décantée, lavée à l'eau, séchée sur sulfate de magnésium et évaporée. Le résidu obtenu est purifié par chromatographie sur colonne de silice, élué avec du dichlorométhane. On évapore les solvants et obtient 2,4g (62%) d'aldéhyde de point de fusion : 243-245°C.

EXEMPLE 17

Préparation de l'alcool 4-[3-(1-adamantyl)-4-méthoxy benzoyloxy]benzylique

2,1g (5,4 mmoles) de l'aldéhyde obtenu à l'exemple 16 sont dissous dans 30ml de méthanol et traités par 310mg (8,2 mmoles) de borohydrure de sodium. Le mélange est agité à température ambiante pendant 2 heures, jeté dans l'eau et extrait à l'éther éthylique. La phase organique est décantée, lavée à l'eau, séchée sur sulfate de magnésium et évaporée. Le résidu ainsi obtenu est purifié par chromatographie sur colonne de silice, en utilisant comme éluant un mélange de dichlorométhane et d'éther éthylique (90/10). On recueille ainsi 1,45g (68%) d'alcool attendu de point de fusion : 168-169°C.

EXEMPLE 18

Préparation de l'acide 3-tert-butyl-4-[3-(1-adamantyl)-4-méthoxybenzoyloxy] benzoïque

(a) 4-hydroxy-3-tert-butylbenzoate d'allyle.

Dans un ballon, on introduit 9,71g (50 mmoles) d'acide 4-hydroxy-3-tert-butylbenzoïque et 55ml d'alcool allylique. On ajoute 1,3ml d'acide sulfurique concentré et chauffe à 100°C durant 18 heures. On évapore à sec, ajoute 200ml d'eau, neutralise avec du bicarbonate de sodium et extrait avec de l'éther éthylique. La phase organique est décantée, lavée à l'eau, séchée sur sulfate de magnésium et évaporée. Le résidu obtenu est purifié par chromatographie sur colonne de silice en éluant avec du dichlorométhane. Après évaporation des solvants, on obtient 9,78g (83%) de l'ester attendu de point de fusion : 131-132°C.

(b) 3-tert-butyl-4-[3-(1-adamantyl)-4-méthoxybenzoyloxy] benzoate d'allyle.

Dans un ballon, on introduit 2,34g (10 mmoles) de l'ester obtenu à l'exemple 18(a), 1,53ml (11 mmoles) de triéthylamine et 30ml de THF. On ajoute goutte à goutte une solution de 3,04g (10 mmoles) de chlorure de l'acide 3-(1-adamantyl)-4-méthoxybenzoïque dans 20ml de THF et agite pendant 24 heures à température ambiante. Le milieu réactionnel est jeté dans l'eau et extrait avec de l'éther éthylique. La phase organique est décantée, lavée à l'eau bicarbonatée puis à l'eau, séchée sur sulfate de magnésium et évaporée. Le résidu obtenu est purifié par chromatographie sur colonne de silice en éluant avec un mélange de dichlorométhane et d'hexane (40-60). On recueille 2,8g (56%) de 3-tert-butyl-4-[3-(1-adamantyl)-4-méthoxybenzoyloxy] benzoate d'allyle de point de fusion : 113-115°C.

(c) Acide-3-tert-butyl-4-[3-(1-adamantyl)-4-méthoxybenzoyloxy] benzoïque.

2,51g (5 mmoles) d'ester préparé à l'exemple 18(b) sont dissous dans 25ml de THF. Sous courant d'azote, on ajoute 145mg de tétrakis (triphénylphosphine) palladium (0) puis introduit goutte goutte 4,4ml (50 mmoles) de morpholine. On agite à température ambiante une heure, évapore à sec, reprend le résidu dans l'éther éthylique et filtre le sel de morpholine formé. On introduit ce sel dans 100ml d'eau, acidifie à PH1 avec de l'acide chlorhydrique concentré et extrait avec de l'éther éthylique. On décante la phase organique, lave à l'eau, sèche sur sulfate de magnésium et évapore. Le résidu est repris dans 50ml d'éther éthylique, filtré et séché. On obtient 1,6g (70%) d'acide attendu de point de fusion : 260-262°C.

EXEMPLES DE FORMULATION

A. VOIE ORALE

(a) comprimé de 0,2g

| | |
|---|---|
| - Acide 4-[3-(1-adamantyl)-4-méthoxybenzoylthio] benzoïque | 0,001g |
| - Amidon | 0,114g |
| - Phosphate bicalcique | 0,020g |
| - Silice | 0,020g |
| - Lactose | 0,030g |
| - Talc | 0,010g |
| - Stéarate de magnésium | 0,005g |

(b) Suspension buvable en ampoules de 5ml

| | |
|---|---|
| - Acide 4-[3-(1-adamantyl)-4-méthoxy benzoyloxy] benzoïque | 0,001g |
| - Glycérine | 0,500g |
| - Sorbitol à 70% | 0,500g |
| - Saccharinate de sodium | 0,010g |
| - Parahydroxybenzoate de méthyle | 0,040g |
| - Arôme qs | |
| - Eau purifiée qsp | 5ml |

(c) comprimé de 0,8g

| | |
|---|---|
| Acide 4-[3-(1-adamantyl)-4-méthoxybenzoyloxy] benzoïque ............................... | 0,500g |
| Amidon prégélatinisé ..................... | 0,100g |
| Cellulose microcristalline ............... | 0,115g |
| Lactose ................................ | 0,075g |
| Stéarate de magnésium .................... | 0,010g |

dans l'exemple (c), l'acide 4- 3-(1-adamantyl)-4-méthoxybenzoyloxy benzoïque, peut être remplacé par l'acide 3-méthyl-4-[3-(1-adamantyl)-4-méthoxybenzoyloxy]benzoïque.

(d) suspension buvable en ampoules de 10ml

| | | |
|---|---|---|
| Acide 4-[3-(1-adamantyl)-4-méthoxybenzoyloxy] benzoïque ................................ | | 0,200g |
| Glycérine ................................ | | 1,000g |
| Sorbitol à 70% ........................... | | 1,000g |
| Saccharinate de sodium ................... | | 0,010g |
| Parahydroxybenzoate de méthyle ............ | | 0,080g |
| Arôme | qs | |
| Eau purifiée | qsp | 10,000ml |

dans l'exemple (d), l'acide 4-[3-(1-adamantyl)-4-méthoxybenzoyloxy] benzoïque peut être remplacé par la même quantité du morpholide de l'acide 4-[3-(1-adamantyl)-4-méthoxybenzoylthio] benzoïque.

B. VOIE TOPIQUE

(a) Onguent

| | |
|---|---|
| - Acide 4-[3-(1-adamantyl)-4-méthoxy benzoylthio]benzoïque.................. | 0,020g |
| - Myristate d'isopropyle................. | 81,700g |
| - Huile de vaseline fluide................ | 9,100g |
| - Silice vendue par la Société DEGUSSA sous le nom "Aérosil 200"............... | 9,180g |

(b) Onguent

| | | |
|---|---|---|
| Acide 4-[3-(1-méthylcyclohexyl)-4-méthoxybenzoylthio] benzoïque .............................. | | 0,300g |
| Vaseline blanche codex | qsp | 100,000g |

dans l'exemple (b), l'acide 4-[3-(1-méthylcyclohexyl)-4-méthoxybenzoylthio]benzoïque peut être remplacé par la même quantité de l'acide 4-[3-(1-adamantyl)-4-méthoxybenzoylthio]benzoïque ou du 4-[-3-(1-adamantyl)-4-méthoxybenzoylthio]benzoate de 2-hydroxyéthyle.

(c) Crème eau-dans-l'huile non ionique

| | |
|---|---|
| Acide 4-[3-(1-adamantyl)-4-hydroxybenzoyloxy] benzoïque .............................. | 0,100g |
| Eucerin anhydre .......................... | 39,900g |
| Parahydroxybenzoate de méthyle ........... | 0,075g |
| Parahydroxybenzoate de propyle ........... | 0,075g |
| Eau déminéralisée stérile qsp | 100,00g |

dans l'exemple (c), l'acide 4-[3-(1-adamantyl)-4-hydroxybenzoyloxy] benzoïque peut être remplacé par la même quantité de l'acide 4-[3-(1-adamantyl)-4-méthoxybenzoylthio] benzoïque.

(d) Crème huile-dans l'eau anionique

| | |
|---|---|
| Acide 4-[3-(1-méthylcyclohexyl)-4-méthoxybenzoylthio] benzoïque ............................... | 1,000g |
| Dodecyl sulfate de sodium ................. | 0,772g |
| 1,2 propanediol .......................... | 1,540g |
| Alcool cétylique ......................... | 19,300g |
| Huile de vaseline épaisse ................ | 19,300g |
| Parahydroxybenzoate de méthyle ........... | 0,075g |
| Parahydroxybenzoate de propyle ........... | 0,075g |
| Eau déminéralisée stérile qsp | 100,000g |

dans l'exemple (d), l'acide 4-[3-(1-méthylcyclohexyl)-4-méthoxybenzoylthio] benzoïque peut être remplacé par la même quantité de l'acide 4-[3-(1-adamantyl)-4-méthoxybenzoylthio] benzoïque

(e) Lotion

| | |
|---|---|
| Acide 4-[3-(1-adamantyl)-4-hydroxybenzoyloxy] benzoïque ............................... | 0,100g |
| PEG 400 ................................ | 69,900g |
| Ethanol 95% ............................ | 30,000g |

dans l'exemple (e), l'acide 4-[3-(1-adamantyl)-4-hydroxybenzoyloxy] benzoïque peut être remplacé par la même quantité de l'acide 4-[3-(1-adamantyl)-4-méthoxybenzoylthio] benzoïque.

(f) Gel éthanolique

| | |
|---|---|
| Acide 4-[3-(1-méthylcyclohexyl)-4-méthoxybenzoylthio] benzoïque ............................... | 0,300g |
| Hydroxypropylcellulose .................. | 2,000g |
| Ethanol 95% qsp | 100,000g |

(g) Onguent hydrophobe

| | |
|---|---|
| Acide 4-[3-(1-adamantyl)-4-méthoxybenzoylthio] benzoate de méthyle ...................... | 0,300g |
| Myristate d'isopropyle ................... | 36,400g |
| Huile de silicone vendue par la Société RHONE-POULENC sous la dénomination Rhodorsil 47 V 300 ... | 36,400g |
| Cire d'abeille ........................... | 13,600g |
| Huile de silicone vendue par la Société GOLDSCHMIDT sous la dénomination Abil 300.000 cst | qsp 100,000g |

(h) Gel aqueux

| | |
|---|---|
| Acide 4-[3-(1-adamantyl)-4-méthoxybenzoylthio] benzoïque ................................ | 0,300g |
| Poloxamer 162 ............................ | 0,200g |
| Propylène glycol ......................... | 4,000g |
| Polymère de l'acide acrylique vendu par la Société GOODRICH sous la dénomination Carbopol 940 | 0,800g |
| EDTA disodique ........................... | 0,100g |
| Parahydroxybenzoate de méthyle ............ | 0,100g |
| NaOH à 10% dans l'eau .................... | 1,250g |
| Eau déminéralisée stérile | qsp 100,000g |

Dans l'exemple (h), l'acide 4-[3-(1-adamantyl)-4-méthoxybenzoylthio] benzoïque peut être remplacé par la même quantité de l'acide 4-[3-(1-adamantyl)-4-méthoxybenzoyloxy] benzoïque.

(i) Gel anhydre

| | |
|---|---|
| Acide 4-[3-(1-adamantyl)-4-méthoxybenzoyloxy] benzoïque ................................ | 0,100g |
| Silice vendue par la Société DEGUSSA sous la dénomination Aérosil 200 .................. | 7,000g |
| Myristate d'isopropyle | qsp 100,000g |

(j) Crème huile-dans-l'eau non ionique

| | |
|---|---|
| Acide 4-[3-(1-adamantyl)-4-méthoxybenzoyloxy] benzoïque .................................. | 1,000g |
| Alcool cétylique .......................... | 4,000g |
| Monostéarate de glycérol .................. | 2,500g |
| Stéarate de PEG 50 ........................ | 2,500g |
| Beurre de Karité .......................... | 9,200g |
| Propylène glycol .......................... | 2,000g |
| Parahydroxybenzoate de méthyle ............ | 0,075g |
| Parahydroxybenzoate de propyle ............ | 0,075g |
| Eau déminéralisée stérile ............. qsp | 100,000g |

(k) Film souple

| | |
|---|---|
| Acide 4-[3-(1-adamantyl)-4-méthoxybenzoylthio] benzoïque ................................ | 0,100g |
| Elastomère silicone vendu par la Société RHONE-POULENC sous la dénomination Rhodorsil RTV 141 A ................................ | 89,900g |
| Catalyseur à froid vendu par la Société RHONE-POULENC sous la dénomination RTV 141 B | 10,000g |

C. ADMINISTRATION EN INJECTABLE

Ampoule injectable par voie intraveineuse

| | |
|---|---|
| Acide 4-[3-(1-adamantyl)-4-hydroxybenzoyloxy] benzoïque micronisé ........................................ | 0,003g |
| Eau pour préparation injectable qsp | 3ml |

dans l'exemple précédent, l'acide 4-[3-(1-adamantyl)-4-hydroxybenzoyloxy] benzoïque peut être remplacé par la même quantité de l'acide 4-[3-(1-adamantyl)-4-hydroxybenzoylthio] benzoïque.

**Revendications**

1. Esters et thioesters aromatiques, caractérisés par le fait qu'ils répondent à la formule générale suivante:

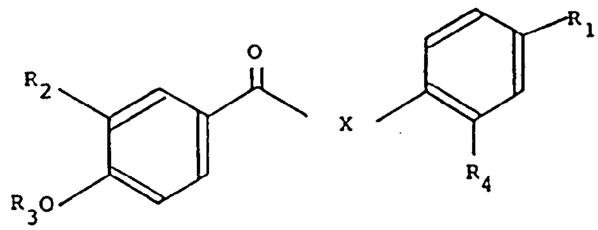

(I)

dans laquelle:

X représente un atome d'oxygène ou de soufre,

$R_1$ représente: $-CH_2OH$ , $-CH(OH)-CH_3$ $-COOR_5$ ou

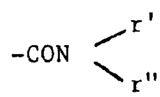

$R_5$ représentant un atome d'hydrogène, un radical alkyle ou alkényle inférieur ou un radical mono ou polyhydroxyalkyle,

r′ et r″ représentant un atome d'hydrogène, un radical alkyle inférieur, un radical mono ou polyhydroxyalkyle, un radical aryle ou benzyle éventuellement substitué(s), un reste d'aminoacide ou de sucre aminé ou pris ensemble forment un hétérocycle,

$R_2$ représente un radical alkyle $\alpha,\alpha'$ disubstitué ayant de 4 à 12 atomes de carbone ou un radical cycloalkyle mono ou polycyclique ayant de 5 à 12 atomes de carbone dont le carbone de liaison est trisubstitué,

$R_3$ représente un atome d'hydrogène, un radical alkyle inférieur, ou le radical $-Si(CH_3)_2R'_3$- $R'_3$représentant un radical alkyle inférieur

et $R_4$ représente un atome d'hydrogène ou un radical alkyle inférieur,

et les sels desdits esters et thioesters aromatiques de formule (I) lorsque $R_5$ représente un atome d'hydrogène.

**2.** Composés selon la revendication 1, caractérisés par le fait que lorsque les composés de formule (I) se présentent sous forme de sels il s'agit de sels d'un métal alcalin ou alcalino-terreux ou encore de zinc ou d'une amine organique.

**3.** Composés selon la revendication 1, caractérisés par le fait que le radical alkyle inférieur a de 1 à 6 atomes de carbone et est pris dans le groupe constitué par les radicaux méthyle, éthyle, isopropyle, butyle et tertiobutyle.

**4.** Composés selon la revendication 1, caractérisés par le fait que le radical alkyle $\alpha$, $\alpha'$ disubstitué est pris dans le groupe constitué par : un radical tert-butyle, 1,1-diméthyl propyle, 1-méthyl 1-éthyl propyle, 1-méthyl 1-éthyl hexyle et 1,1-diméthyl décyle.

**5.** Composés selon la revendication 1, caractérisés par le fait que le radical monohydroxyalkyle est un radical 2-hydroxyéthyle, 2-hydroxypropyle ou 3-hydroxypropyle.

**6.** Composés selon la revendication 1, caractérisés par le fait que le radical polyhydroxyalkyle comporte de 3 à 6 atomes de carbone et de 2 à 5 groupes hydroxyles et est pris dans le groupe constitué par le radical 2,3-dihydroxypropyle, 2,3,4-trihydroxybutyle, 2,3,4,5-tétrahydroxy pentyle et le reste du pentaérythritol.

**7.** Composés selon la revendication 1, caractérisés par le fait que le radical aryle est un radical phényle éventuellement substitué par un atome d'halogène, un hydroxyle ou une fonction nitro.

**8.** Composés selon la revendication 1, caractérisés par le fait que le radical cycloalkyle mono ou polycyclique ayant de 5 à 12 atomes de carbone dont le carbone de liaison est trisubstitué est le radical 1-méthylcyclohexyle ou 1-adamantyle.

**9.** Composés selon la revendication 1, caractérisés par le fait que le reste d'amino acide est un reste dérivant de lysine ou de glycine.

10. Composés selon la revendication 1, caractérisés par le fait que le reste d'un sucre aminé est un reste dérivant de glucosamine, de galactosamine ou de mannosamine.

11. Composés selon la revendication 1, caractérisés par le fait que les radicaux r' et r" pris ensemble forment un hétérocycle pris dans le groupe constitué par un radical pipéridino, pipérazino, morpholino, pyrrolidino ou (2-hydroxyéthyl)-4 pipérazino.

12. Composés selon l'une quelconque des revendications précédentes, caractérisés par le fait qu'ils sont pris dans le groupe constitué par:
- l'acide 4-[3-[1-adamantyl)-4-méthoxybenzoyloxy] benzoïque,
- l'acide 4-[3-(1-adamantyl)-4-méthoxybenzoylthio] benzoïque,
- l'acide 4-[3-(1-méthylcyclohexyl)-4-méthoxybenzoyloxy] benzoïque,
- l'acide 4-[3-(1-méthylcyclohexyl)-4-méthoxybenzoylthio] benzoïque,
- l'acide 4-[3-(1,1-diméthyldécyl)-4-méthoxybenzoyloxy] benzoïque,
- l'acide 4-[3-(1,1-diméthyldécyl)-4-méthoxybenzoylthio] benzoïque,
- l'acide 4-[3-(1-adamantyl)-4-hydroxybenzoyloxy] benzoïque,
- l'acide 4-[3-(1-adamantyl)-4 hydroxybenzoylthio] benzoïque,
- le 4-[3-(1-adamantyl)-4-méthoxybenzoyloxy] benzoate d'éthyle,
- le 4-[3-(1-adamantyl)-4-méthoxybenzoylthio] benzoate d'éthyle,
- le N-éthyl 4-[3-(1-adamantyl)-4-méthoxybenzoyloxy] benzamide,
- le morpholide de l'acide 4-[3-(1-adamantyl)-4-méthoxybenzoylthio] benzoïque,
- le 4-[3-(1-adamantyl)-4-méthoxybenzoyloxy] benzoate d'allyle.
- l'acide 4-[3-tert-butyl-4-méthoxybenzoylthio] benzoïque,
- le 4-[3-(1-adamantyl)-4-méthoxybenzoylthio] benzoate de méthyle,
- Le N-éthyl 4-[3-(1-adamantyl)-4-méthoxybenzoylthio] benzamide,
- le 4-[3-(1-adamantyl)-4-méthoxybenzoylthio] benzoate de 2-hydroxyéthyle,
- l'acide 4-[3-(1-adamantyl)-4-tert-butyldiméthylsilyloxy benzoylthio] benzoïque,
- le 4-[3-(1-adamantyl)-4-hydroxybenzoyloxy] benzoate d'allyle,
- l'acide 3-méthyl-4-[3-(1-adamantyl)-4-méthoxybenzoyloxy] benzoïque,
- l'aldéhyde 4-[3-(1-adamantyl)-4-méthoxybenzoyloxy] benzoïque,
- l'alcool 4-[3-(1-adamantyl)-4-méthoxybenzoyloxy] benzylique, et
- l'acide 3-tert-butyl-4-[3-(1-adamantyl)-4-méthoxybenzoyloxy] benzoïque.

13. Procédé de préparation des composés de formule (I) selon l'une quelconque des revendications 1 à 12, caractérisé par le fait qu'il consiste à faire réagir, dans un solvant organique, en présence d'une amine tertiaire, une forme activée d'un acide benzoïque substitué notamment un chlorure d'acide de formule:

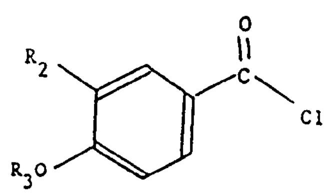

sur un acide p-hydroxy ou p-thio benzoïque de formule:

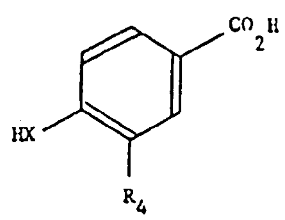

dans lesquelles :

X, et $R_2$ , $R_3$ et $R_4$ ont les mêmes significations que celles données à la revendication 1, la réaction étant conduite à température ambiante sous agitation, et que l'on procède si nécessaire à la formation du chlorure d'acide correspondant et à la transformation subséquente dudit chlorure soit en ester par ac-

tion d'un alcool ($R_5OH$) soit en amide par action d'une amine

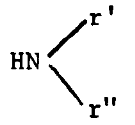

**14.** Procédé de préparation des composés de formule (I) selon l'une quelconque des revendications 1 à 12, dans laquelle X représente un atome d'oxygène, caractérisé par le fait qu'il consiste à faire réagir, en présence d'une amine tertiaire, un chlorure d'acide de formule:

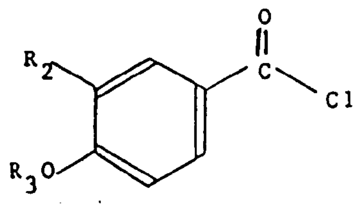

sur un p-hydroxybenzoate d'allyle de formule:

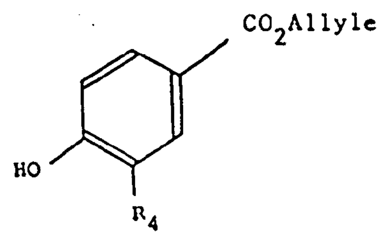

dans lesquelles:

$R_2$ $R_3$ et $R_4$ ont les mêmes significations que celles données à la revendication 1, que l'on transforme l'ester allylique obtenu en acide correspondant en présence, comme catalyseur, d'un complexe de métaux de transition, et que l'on procède, si nécessaire, à la formation du chlorure d'acide correspondant et à la transformation subséquente dudit chlorure soit en ester par action d'un alcool ($R_5OH$) soit en amide par action d'une amine

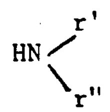

**15.** Composition pharmaceutique, caractérisée par le fait qu'elle contient dans un véhicule approprié, pour une administration par voie entérale, parentérale, topique ou oculaire, au moins un composé de formule (I) selon l'une quelconque des revendication 1 à 12.

**16.** Composition selon la revendication 15, caractérisée par le fait qu'elle contient de 0,0001 à environ 5% en poids d'un composé de formule (I).

**17.** Utilisation d'un composé selon l'une quelconque des revendications 1 à 12, pour la préparation d'une composition pharmaceutique destinée au traitement des affections dermatologiques, rhumatismales, respiratoires ainsi qu'ophtalmologiques.

**18.** Composition cosmétique pour l'hygiène corporelle et capillaire, caractérisée par le fait qu'elle contient, dans un véhicule cosmétique approprié, au moins un composé de formule (I) selon l'une quelconque des revendications 1 à 12.

**19.** Composition cosmétique selon la revendication 18, caractérisée par le fait qu'elle contient le composé

de formule (I) à une concentration comprise entre 0,0001 et 0,1% et de préférence entre 0,001 et 0,01% en poids.

## Patentansprüche

1. Aromatische Ester und Thioester, dadurch gekennzeichnet, daß sie der folgenden allgemeinen Formel entsprechen:

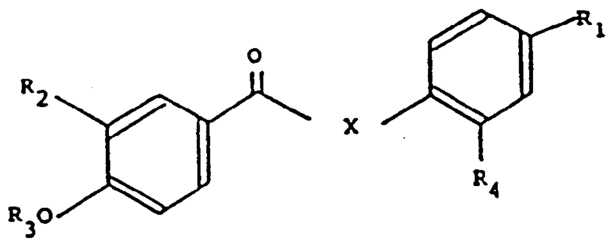

(I)

in der

X für ein Sauerstoff- oder Schwefel-Atom steht;

$R_1$ für folgende Gruppen steht: $-CH_2OH$, $-CH(OH)-CH_3$, $-COOR_5$ oder

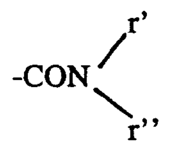

$R_5$ für ein Wasserstoff-Atom, einen niederen Alkyl-Rest oder Alkenyl-Rest oder einen Monohydroxyalkyl-Rest oder Polyhydroxyalkyl-Rest steht;

r' und r" für ein Wasserstoff-Atom, einen niederen Alkyl-Rest, einen Monohydroxyalkyl-Rest oder Polyhydroxyalkyl-Rest, einen gegebenenfalls substituierten Aryl-Rest oder Benzyl-Rest, einen Aminosäure-Rest oder Rest eines aminierten Zuckers stehen oder zusammengenommen einen Heterozyklus bilden;

$R_2$ einen α, α'-disubstituierten Alkyl-Rest, der 4 bis 12 Kohlenstoff-Atome aufweist, oder einen monozyklischen oder polyzyklischen Cycloalkyl-Rest bedeutet, der 5 bis 12 Kohlenstoff-Atome aufweist und in dem der Verbindungs-Kohlenstoff dreifach substituiert ist;

$R_3$ für ein Wasserstoff-Atom, einen niederen Alkyl-Rest oder den Rest $-Si(CH_3)_2R'_3$ steht, worin $R'_3$ einen niederen Alkyl-Rest bedeutet; und

$R_4$ für ein Wasserstoff-Atom oder einen niederen Alkyl-Rest steht;

und die Salze dieser aromatischen Ester und Thioester der Formel (I), wenn $R^5$ für ein Wasserstoff-Atom steht.

2. Verbindungen nach Anspruch 1, dadurch gekennzeichnet, daß dann, wenn die Verbindungen der Formel (I) in Form von Salzen vorliegen, es sich um Salze eines Alkalimetalls oder Erdalkalimetalls oder auch des Zinks oder eines organischen Amins handelt.

3. Verbindungen nach Anspruch 1, dadurch gekennzeichnet, daß der niedere Alkyl-Rest 1 bis 6 Kohlenstoff-Atome aufweist und gewählt ist aus der Methyl-Resten, Ethyl-Resten, Isopropyl-Resten, Butyl-Resten und tert. -Butyl-Resten bestehenden Gruppe.

4. Verbindungen nach Anspruch 1, dadurch gekennzeichnet, daß der α, α'-disubstituierte Alkyl-Rest gewählt ist aus der aus tert. -Butyl-Rest, 1, 1-Dimethylpropyl-Rest, 1-Methyl-1-ethyl-propyl-Rest, 1-Methyl-1-ethyl-hexyl-Rest und 1,1-Dimethyldecyl-Rest bestehenden Grupppe.

5. Verbindungen nach Anspruch 1, dadurch gekennzeichnet, daß der Monohydroxyalkyl-Rest ein 2-Hydroxyethyl-Rest, 2-Hydroxypropyl-Rest oder 3-Hydroxypropyl-Rest ist.

6. Verbindungen nach Anspruch 1, dadurch gekennzeichnet, daß der Polyhydroxyalkyl-Rest 3 bis 6 Kohlenstoff-Atome und 2 bis 5 Hydroxy-Gruppen umfaßt und gewählt ist aus der aus 2,3-Dihydroxypropyl-Rest, 2,3,4,-Trihydroxybutyl-Rest, 2,3,4,5-Tetrahydroxypentyl-Rest und Pentaerythrit(ol)-Rest bestehenden Gruppe.

7. Verbindungen nach Anspruch 1, dadurch gekennzeichnet, daß der Aryl-Rest ein Phenyl-Rest ist, der gegebenenfalls durch ein Halogen-Atom, eine Hydroxy-Gruppe oder eine Nitrofunktion substituiert ist.

8. Verbindungen nach Anspruch 1, dadurch gekennzeichnet, daß der monozyklische oder polyzyklische Cycloalkyl-Rest mit 5 bis 12 Kohlenstoff-Atomen, in dem das Verbindungs-Kohlenstoff-Atom trisubstituiert ist, der 1-Methylcyclohexyl-Rest oder 1-Adamantyl-Rest ist.

9. Verbindungen nach Anspruch 1, dadurch gekennzeichnet, daß der Aminosäure-Rest ein Rest ist, der von Lysin oder Glycin abgeleitet ist.

10. Verbindungen nach Anspruch 1, dadurch gekennzeichnet, daß der Rest eines aminierten Zuckers ein Rest ist, der von Glucosamin, Galactosamin oder Mannosamin abeleitet ist.

11. Verbindungen nach Anspruch 1, dadurch gekennzeichnet, daß die Reste r' und r" zusammengenommen einen Heterozyklus bilden, der aus der aus Piperidino-Rest, Piperazino-Rest, Morpholino-Rest, Pyrrolidino-Rest oder (2-Hydroxyethyl-)4-piperazino-Rest bestehenden Gruppe gewählt ist.

12. Verbindungen nach irgendeinem der vorangehenden Ansprüche, dadurch gekennzeichnet, daß sie aus der Gruppe folgender Verbindungen ausgewählt sind:
4-[3-(1-Adamantyl-)4-methoxybenzoyloxy-]benzoesäure;
4-[3-(1-Adamantyl-)4-methoxybenzoylthio-]benzoesäure;
4-[3-(1-Methylcyclohexyl-)4-methoxybenzoyloxy-]benzoesäure;
4-[3-(1-Methylcyclohexyl-)4-methoxybenzoylthio-]benzoesäure;
4-[3-(1,1-Dimethyldecyl-)4-methoxybenzoyloxy-]benzoesäure;
4-[3-(1,1-Dimethyldecyl-)4-methoxybenzoylthio-]benzoesäure;
4-[3-(1-Adamantyl-)4-hydroxybenzoyloxy-]benzoesäure;
4-[3-(1-Adamantyl-)4-hydroxybenzoylthio-]benzoesäure;
4-[3-(1-Adamantyl-)4-methoxybenzoyloxy-]benzoesäureethylester;
4-[3-(1-Adamantyl-)4-methoxybenzoylthio-]benzoesäureethylester;
N-Ethyl-4-[3-(1-adamantyl-)4-methoxybenzoyloxy-]benzamid;
4-[3-(1-Adamantyl-)4-methoxybenzoylthio-]benzoesäuremorpholid;
4-[3-(1-Adamantyl-)4-methoxybenzoyloxy-]benzoesäureallylester;
4-[3-(tert.-Butyl-)4-methoxybenzoylthio-]benzoesäure
4-[3-(1-Adamantyl-)4-methoxybenzoylthio-]benzoesäuremethylester
N-Ethyl-4-[3-(1-adamantyl-)4-methoxybenzoylthio-]benzamid
4-[3-(1-Adamantyl-)4-methoxybenzoylthio-]benzoesäure-2-hydroxyethylester;
4-[3-(1-Adamantyl-)4-tert.-butyldimethylsilyloxybenzoylthio-]benzoesäure;
4-[3-(1-Adamantyl-)4-hydroxybenzoyloxy-]benzoesäureallylester;
3-Methyl-4-[3-(1-adamantyl-)4-methoxybenzoyloxy-]benzoesäure;
4-[3-(1-Adamantyl-)4-methoxybenzoyloxy-]benzaldehyd;
4-[3-(1-Adamantyl-)4-methoxybenzoyloxy-]benzylalkohol; und
3-tert.-Butyl-4-[3-(1-adamantyl-)4-methoxybenzoyloxy-]benzoesäure.

13. Verfahren zur Herstellung der Verbindungen der Formel (I) nach irgendeinem der Ansprüche 1 bis 12, dadurch gekennzeichnet, daß es daraus besteht, in einem organischen Lösungsmittel in Gegenwart eines tertiären Amins eine aktivierte Form einer substituierten Benzoesäure, insbesondere ein Säurechlorid der Formel

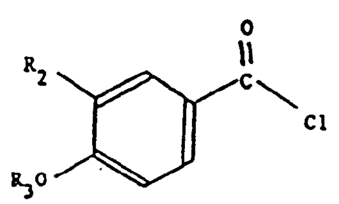

mit einer p-Hydroxy- oder p-Thio-Benzoesäure der Formel

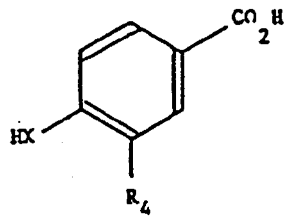

umzusetzen, in denen

X, $R_2$, $R_3$ und $R_4$ die gleichen Bedeutungen wie die haben, die in Patentanspruch 1 angegeben sind, wobei die Reaktion bei Umgebungstemperatur unter Rühren durchgeführt wird, und daß man anschließend - sofern erforderlich - das Chlorid der entsprechenden Säure herstellt und nachfolgend das Säurechlorid entweder durch Einwirkung eines Alkohols $R_5OH$ in einen Ester oder durch Einwirkung eines Amins

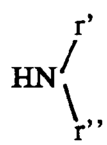

in ein Amid überführt.

**14.** Verfahren zur Herstellung von Verbindungen der Formel (I) nach irgendeinem der Patentansprüche 1 bis 12, worin X für ein Sauerstoff-Atom steht, dadurch gekennzeichnet, daß das Verfahren daraus besteht, daß man in Gegenwart eines tertiären Amins ein Säurechlorid der Formel

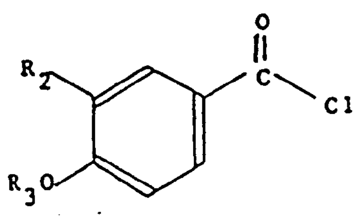

mit einem p-Hydroxybenzoesäureallylester der Formel

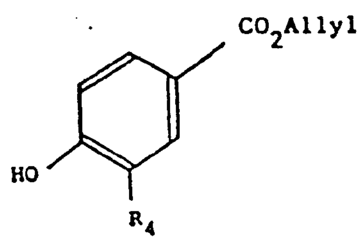

umsetzt, worin

$R_2$, $R_3$ und $R_4$ die gleichen Bedeutungen haben, wie sie in Patentanspruch 1 angegeben sind, daß man den erhaltenen Allylester in Gegenwart eines Übergangsmetall-Komplexes als Katalysator in die entsprechende Säure umwandelt, und daß man - sofern erforderlich - das Chlorid der entsprechenden Säure herstellt und danach das Chlorid entweder durch Einwirkung eines Alkohols $R_5OH$ in einen Ester oder durch Einwirkung eines Amins

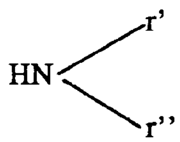

in ein Amid umwandelt.

**15.** Pharmazeutische Zubereitung, dadurch gekennzeichnet, daß sie in einem geeigneten Träger zur enteralen, parenteralen oder topischen Verabreichung oder zur Verabreichung in die Augen wenigstens eine Verbindung der Formel (I) nach irgendeinem der Patentansprüche 1 bis 12 enthält.

**16.** Zubereitung nach Anspruch 15, dadurch gekennzeichnet, daß sie 0,0001 bis etwa 5 Gew. - % einer Verbindung der Formel (I) enthält.

**17.** Verwendung einer Verbindung gemäß irgendeinem der Ansprüche 1 bis 12 zur Herstellung einer pharmazeutischen Zubereitung, die bestimmt ist zur Behandlung von dermatologischen, rheumatischen, Atmungs- sowie ophtalmologischen Erkrankungen.

**18.** Kosmetische Zubereitung zur Körper- und Haar-Hygiene, dadurch gekennzeichnet, daß sie in einem geeigneten kosmetischen Träger wenigstens eine Verbindung der Formel (I) nach irgendeinem der Ansprüche 1 bis 12 enthält.

**19.** Kosmetische Zubereitung nach Anspruch 18, dadurch gekennzeichnet, daß sie die Verbindung der Formel (I) in einer Konzentration zwischen 0,0001 und 0,1 Gew.-% einschließlich enthält, vorzugsweise zwischen 0,001 und 0,01 Gew.-%.

**Claims**

**1.** Aromatic esters and thioesters, characterised in that they correspond to the following general formula:

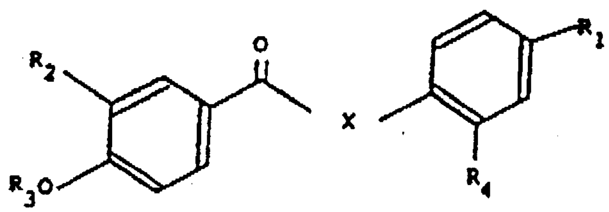

(I)

in which :

X represents an oxygen or sulphur atom,

$R_1$ represents: $-CH_2OH$, $-CH(OH)-CH_3$, $-COOR_5$ or

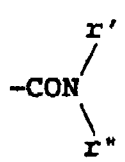

$R_5$ representing a hydrogen atom, a lower alkyl or alkenyl radical or a mono- or polyhydroxyalkyl radical,

R′ and R″ representing a hydrogen atom, a lower alkyl radical, a mono- or polyhydroxyalkyl radical, an optionally substituted aryl or benzyl radical, an amino acid or amino sugar residue or, taken together, form a heterocycle,

$R_2$ represents an α, α′-disubstituted alkyl radical having from 4 to 12 carbon atoms or a mono- or polycyclic cycloalkyl radical having from 5 to 12 carbon atoms, the linking carbon of which is trisubstituted,

$R_3$ represents a hydrogen atom, a lower alkyl radical or the radical $-Si(CH_3)_2R'_3$, $R'_2$ representing a lower alkyl radical

and $R_4$ represents a hydrogen atom or a lower alkyl radical,

and the salts of the said aromatic esters and thioesters of formula (I) when $R_5$ represents a hydrogen atom.

2. Compounds according to Claim 1, characterised in that when the compounds of formula (I) are in the form of salts, they are salts of an alkali metal or alkaline earth metal or also of zinc or of an organic amine.

3. Compounds according to Claim 1, characterised in that the lower alkyl radical has from 1 to 6 carbon atoms and is taken from the group consisting of the methyl, ethyl, isopropyl, butyl and tert-butyl radicals.

4. Compounds according to Claim 1, characterised in that the α, α'-disubstituted alkyl radical is taken from the group consisting of: a tert-butyl, 1,1-dimethylpropyl, 1-methyl-1-ethylpropyl, 1-methyl-1-ethylhexyl and 1,1-dimethyldecyl radical.

5. Compounds according to Claim 1, characterised in that the monohydroxyalkyl radical is a 2-hydroxyethyl, 2-hydroxypropyl or 3-hydroxypropyl radical.

6. Compound according to Claim 1, characterised in that the polyhydroxyalkyl radical contains from 3 to 6 carbon atoms and from 2 to 5 hydroxyl groups and is taken from the group consisting of the 2,3-dihydroxypropyl, 2,3,4-trihydroxybutyl or 2,3,4,5-tetrahydroxypentyl radical and the pentaerythritol residue.

7. Compounds according to Claim 1, characterised in that the aryl radical is a phenyl radical, optionally substituted with a halogen atom, a hydroxyl group or a nitro functional group.

8. Compounds according to Claim 1, characterised in that the mono- or polycyclic cycloalkyl radical having from 5 to 12 carbon atoms, the linking carbon of which is trisubstituted, is the 1-methylcyclohexyl or 1-adamantyl radical.

9. Compounds according to Claim 1, characterised in that the amino acid residue is a residue deriving from lysine or glycine.

10. Compounds according to Claim 1, characterised in that the residue of an amino sugar is a residue deriving from glucosamine, galactosamine or mannosamine.

11. Compounds according to Claim 1, characterised in that the r' and r'' radicals, taken together, form a heterocycle taken from the group consisting of a piperidino, piperazino, morpholino, pyrrolidino or 4-(2-hydroxyethyl)piperazino radical.

12. Compounds according to any one of the above claims, characterised in that they are taken from the group consisting of:
- 4-[3-(1-adamantyl)-4-methoxybenzoyloxy]benzoic acid,
- 4-[3-(1-adamantyl)-4-methoxybenzoylthio]benzoic acid,
- 4-[3-(1-methylcyclohexyl)-4-methoxybenzoyloxy]benzoic acid,
- 4-[3-(1-methylcyclohexyl)-4-methoxybenzoylthio]benzoic acid,
- 4-(3-(1,1-dimethyldecyl)-4-methoxybenzoyloxy]benzoic acid,
- 4-(3-(1,1-dimethyldecyl)-4-methoxybenzoylthio]benzoic acid,
- 4-[3-(1-adamantyl)-4-hydroxybenzoyloxy]benzoic acid,
- 4-[3-(1-adamantyl)-4-hydroxybenzoylthio]benzoic acid,
- ethyl 4-[3-(1-adamantyl)-4-methoxybenzoyloxy]benzoate,
- ethyl 4-[3-(1-adamantyl)-4-methoxybenzoylthio]benzoate,
- N-ethyl-4-[3-(1-adamantyl)-4-methoxybenzoyloxy]benzamide,
- the morpholide of 4-[3-(1-adamantyl)-4-methoxybenzoylthio]benzoic acid,
- allyl 4-[3-(1-adamantyl)-4-methoxybenzoyloxy]benzoate,
- 4-[3-(tert-butyl)-4-methoxybenzoylthio]benzoic acid,
- methyl 4-[3-(1-adamantyl)-4-methoxybenzoylthio]benzoate,
- N-ethyl-4-[3-(1-adamantyl)-4-methoxybenzoylthio]benzamide,
- 2-hydroxyethyl 4-[3-(1-adamantyl)-4-methoxybenzoyltio]benzoate,

- 4-[3-(1-adamantyl)-4-(tert-butyldimethylsilyloxy)benzoylthio]benzoic acid,
- allyl 4-[3-(1-adamantyl)-4-hydroxybenzoyloxy]benzoate,
- 3-methyl-4-[3-(1-adamantyl)-4-methoxybenzoyloxy]benzoic acid,
- 4-[3-(1-adamantyl)-4-methoxybenzoyloxy]benzaldehyde,
- 4-[3-(1-adamantyl)-4-methoxybenzoyloxy]benzyl alcohol, and
- 3-tert-butyl-4-[3-(1-adamantyl)-4-methoxybenzoyloxy]benzoic acid,

**13.** Process for the preparation of compounds of formula (I) according to any one of Claims 1 to 12, characterised in that it consists in reacting, in an organic solvent, in the presence of a tertiary amine, an activated form of a substituted benzoic acid, especially an acid chloride of formula:

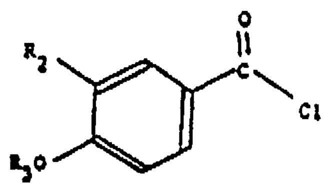

with a p-hydroxy- or p-thiobenzoic acid of formula:

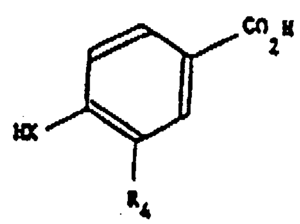

in which:

X, $R_2$, $R_3$ and $R_4$ have the same meanings as those given in Claim 1, the reaction being carried out at room temperature with stirring, and in that, if necessary, the formation of the corresponding acid chloride and the subsequent conversion of the said chloride either to an ester, by reaction with an alcohol ($R_5OH$), or to an amide, by reacting with an amine

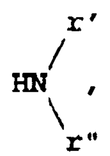

are carried out.

**14.** Process for the preparation of the compounds of formula (I) according to any one of Claims 1 to 12, in which X represents an oxygen atom, characterised in that it consists in reacting, in the presence of a tertiary amine, an acid chloride of formula:

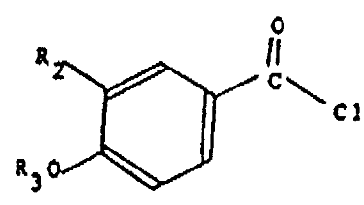

with an allyl p-hydroxybenzoate of formula:

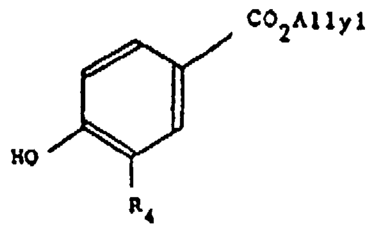

in which:

$R_2$, $R_3$ and $R_4$ have the same meanings as those given in Claim 1, in that the allyl ester obtained is converted into the corresponding acid in the presence, as catalyst, of a transition metal complex, and in that, if necessary, the formation of the corresponding acid chlorid and the subsequent conversion of the said chloride either to an ester, by reacting with an alcohol ($R_5OH$), or to an amide, by reacting with an amine

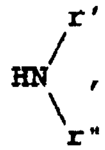

are carried out.

**15.** Pharmaceutical composition, characterised in that it contains in a suitable vehicle, for enteral, parenteral, topical or ocular administration, at least one compound of formula (I) according to any one of Claims 1 to 12.

**16.** Composition according to Claim 15, characterised in that it contains from 0.0001 to approximately 5% by weight of a compound of formula (I).

**17.** Use of a compound according to any one of Claims 1 to 12, for the preparation of a pharmaceutical composition intended for the treatment of dermatological, rheumatic, respiratory and ophthalmological complaints.

**18.** Cosmetic composition for body and hair hygiene, characterised in that it contains, in a suitable cosmetic vehicle, at least one compound of the formula (I) according to any one of Claims 1 to 12.

**19.** Cosmetic composition according to Claim 18, characterised in that it contains the compound of formula (I) at a concentration of between (0.0001 and 0.1% and preferably between 0.001 and 0.01% by weight.